# EUROPÄISCHE PATENTSCHRIFT

(11) **EP 1 834 625 B1**
(45) Veröffentlichungstag und Bekanntmachung des Hinweises auf die Patenterteilung: **26.05.2010**
(21) Anmeldenummer: 07004688.3
(22) Anmeldetag: 07.03.2007
(51) Int. Cl.: A61J 7/00, B65D 25/06

(54) **Modul-Wannensystem**
Module tank system
Système de cuve modulaire

(30) Priorität: 15.03.2006 DE 20604099 U
(43) Veröffentlichungstag der Anmeldung: 19.09.2007
(73) Patentinhaber: H+H SYSTEM GmbH, 5350 Strobl / Wolfgangsee (AT)
(72) Erfinder: Hrovat jun., Heimo, Mag.pharm., 4820 Bad Ischl (AT)
(74) Vertreter: Laufhütte, Dieter

(56) Entgegenhaltungen:
- DE-A1- 10 224 588
- FR-A- 1 073 514
- FR-A- 2 697 503
- FR-A1- 2 235 847
- FR-A1- 2 247 179
- US-A- 2 108 950

## Beschreibung

Die Erfindung betrifft ein Modul-Wannensystem, insbesondere zur Lagerung von Medikamenten und Verbrauchsgütern für medizinische Zwecke.

Derartige Modul-Wannensysteme finden im großen Umfang im Gesundheitsweisen, beispielsweise in Krankenhäusern und Altenheimen Verwendung. Modul-Wannensysteme bestehen üblicherweise aus einer einen Boden und Seitenwände aufweisenden Wanne und in die Wanne senkrecht zum Boden verstellbar einsetzbare Teiler. Die Wanne kann auch in Form von Körben ausgebildet sein. Die üblichen Modulgrößen sind nach ISO oder DIN genormt. Nach ISO-Norm haben die Module eine Größe von 60 x 40 cm, während die DIN-Norm Abmessung 54 x 50 cm beträgt. Es werden auch entsprechende Modul-Wannensysteme in Sondergrößen angeboten. Die Modul-Wannensysteme besitzen in der Regel flanschartige Ränder und werden in Trägerwände mit Schlitzen waagerecht oder schräg eingeschoben. Über entsprechende Stopper sind sie vor dem Herausfallen gesichert. Die Trägerwände in dem Modul-Wannensystem können beispielsweise aus recycelfähigem Acrylnitryl/Butadien/Styrolpolymer gefertigten Formteilen bestehen, die in Schränken, wie Hochschränken, Unterbauschränken oder Hängeschränken, Regalen und/oder Transportwagen beidseitig angeschraubt werden.

Die Modul-Wannensysteme und/oder Körbe werden aus verschiedenen Kunststoffen tiefgezogen bzw. gespritzt oder aus rilsanierten Draht hergestellt. Die Wannen weisen im Bereich ihrer Seitenwände innen Schlitze oder vorstehende Elemente auf, in die die Teiler einsteckbar sind. Ein Hauptnachteil dieser Schlitze besteht darin, dass sich in diesen Schmutz ansammelt, der nur schwer wieder entfernt werden kann. Bei Körben werden die Teiler auf entsprechende Stege der Körbe aufgeclipst oder gesteckt. Die entsprechend in den Seitenwänden gehaltenen Teiler können bei einer Belastung beispielsweise durch schwerere im Modul-Wannensystem gelagerte Teile verbogen und so in unerwünschter Art und Weise aus dem Schlitzbereich in der Seitenwand herausgedrückt werden.

Soweit im Bereich der Seitenwand keine Schlitze ausgeführt sind, können sogenannte Ladeneinsätze in das Modul hineingestellt werden, die bei Bedarf herausgenommen werden können. Hier handelt es sich um einen Kunststoffrahmen, in den Längsschienen und Querteiler, die gegebenenfalls variabel verstellbar sein können, eingesetzt sind. Diese Ausführungsform hat jedoch den Nachteil, dass der umlaufende Kunststoffrahmen, der benachbart zu den Seitenwänden der Wanne liegt, unnötig Platz benötigt. Wenn aufgrund der Hygiene und insbesondere der besseren Variabilität auch glatte Wände grundsätzlich bevorzugt werden, ist die Verwendung des vorgenannten Ladeneinsatzes aufgrund dieses Platzverlustes nachteilig.

DE 102 245 88 offenbart ein Modul-Wannensystem mit aller technischen Merkmalen des Oberbegriffs des Anspruchs 1.

Aufgabe der Erfindung ist es daher, ein Modul-Wannensystem zu schaffen, bei dem die Wannen aus einem Boden und Seitenwänden bestehen, die glatt ausgeführt sind, wobei senkrecht zum Boden verstellbar Teiler in einer feinen Rasterung einstellbar einsetzbar sind, wobei der Platz des Modul-Wannensystems möglichst weitgehend für die Lagerung des Lagerungsgutes ausgenutzt werden kann.

Erfindungsgemäß wird diese Aufgabe durch die Kombination der Merkmale des Anspruchs 1 gelöst.

In besonders vorteilhafter Art und Weise können sowohl die Ausführungen als Wanne wie auch als baugleiche Teiler ausgestattet werden.

Bevorzugte Ausgestaltungen der Erfindung ergeben sich aus den sich an den Hauptanspruch anschließenden Unteransprüchen.

Demnach ist vorteilhaft im Steckclip ein rastbarer Federmechanismus integriert. Der rastbare Federmechanismus kann dabei mindestens einen Widerhaken umfassen, der in der Lochung verrastbar ist.

Somit kann der Steckclip vom Anwender bei entsprechendem Entrasten des Federmechanismus aus seiner Position nach oben abgezogen werden. Umgekehrt kann er nach Einstecken mittels des Steckstifts und der Widerhaken in die jeweilige Lochung in einfacher Weise fest verrastet werden.

Dabei ist es von weiterem Vorteil, wenn der Steckclip einen die Seitenwand übergreifenden Bügel aufweist. Hierdurch wird eine stabile Halterung des Steckclips in alle Richtungen erreicht.

Vorteilhaft ist an jedem Ende des Teilers ein Steckclip angeordnet. Hierdurch lässt sich eine besonders feste Verankerung des Teilers erreichen.

Steckclip und Teilersind mehrteilig und zusammensteckbar ausgebildet. Am Steckclip ist ein U-förmiger Bügel zur Aufnahme des Teilers ausgebildet ist. Zur besseren Verbindung zwischen Teiler- und Steckclip kann innerhalb des U-förmigen Bügels mindestens ein Stift ausgebildet sein, der in aufgesteckter Position in mindestens eine entsprechende Bohrung im Teiler eingreift. Hierdurch wird eine stabile Verbindung zwischen Teiler und Steckclip erreicht.

Gemäß einer besonders vorteilhaften Ausgestaltung der Erfindung kann die Lochung im Boden der Wanne angeordnet sein. Ein Vorteil dieser Ausführungsvariante ist es, dass die Wanne in einer Waschanlage in einfacher Weise gereinigt werden kann. Der in den Bohrungen sich ansammelnde Schmutz würde in einer Waschanlage entsprechend durch die Bohrung hindurchfallen.

Möchte man allerdings eine auslaufsichere Wanne schaffen, kann die Lochung auch in einem Absatz der Seitenwandung angeordnet sein. In diesem Fall wäre die Bodenkante dicht ausgebildet, so dass keine Flüssigkeit auslaufen kann.

Eine dritte Möglichkeit der Anordnung der Lochung bestünde darin, diese am oberen Rand der Wanne anzuordnen. Hier müßte der Clip mit den entsprechenden Widerhaken entsprechend ausgeformt sein, um hier ein Eingreifen der Widerhaken in die jeweilige Lochung zu gewährleisten.

In den jeweiligen Bohrlöchern ist vorteilhaft ein Hinterschnitt zur Aufnahme der Rasthaken des rastbaren Federmechanismus der Steckclips vorgesehen. Hierdurch wird sichergestellt, dass die jeweiligen Rasthaken nicht nach unten aus den Bohrungen hervorstehen.

Das Modul-Wannensystem der vorliegenden Erfindung kann erfindungsgemäß in unterschiedlichen Höhen bereitgestellt werden. So bietet es sich an, bei einem vergleichsweise flacheren Modul-Wannensystem die Bohrungen im Boden vorzusehen, während bei einem vergleichsweise höheren Modul-Wannensystem die Bohrungen in einem Absatz in der Seitenwand angeordnet werden können. Dabei können die Modulgrößen so gewählt sein, dass in jedem Fall Steckclips gleicher Baugröße verwendbar sind.

Gemäß einer weiteren Ausgestaltung der Erfindung kann die Wanne einen flanschartigen Rand aufweisen, der im Randbereich der Wanne unter Ausbildung einer durch den Bügel des Steckclips übergreifbaren Wandung nach unten zurückspringt. Über diesen flanschartigen Rand werden die Wannen üblicherweise in den Seitenwänden des Regals, des Schranks oder des Transportwagens gehalten.

Die Wannen des Modul-Wannensystems können entweder geschlossen oder auch mit Durchbrüchen in Form von Körben ausgebildet sein.

In einer Wanne kann beispielsweise ein Längsteiler über zwei Steckclips gehalten sein, auf dem wiederum senkrecht zu diesem ausgerichtete Schiebeteiler sitzen. Diese Ausführung ermöglicht es, die gesamte Unterteilung durch Herausnehmen des Schiebteilers aus der Wanne zu entfernen und bei Bedarf wieder einzusetzen. Aufgrund dieser Ausführung kann die Wanne beispielsweise in einfacher Art und Weise gereinigt werden, ohne dass hier die gesamten Schiebeteiler einzeln herausgenommen und vor dem Einsetzen wieder zusammengesetzt werden müssen.

In einer Wanne können mehrere parallel zueinander angeordnete Teiler eingesetzt sein. An den Teilern können an sich bekannte senkrecht zu diesen ausgerichtete Schiebeteiler sitzen.

Alternativ können senkrecht zu den Teilern andere Teiler über jeweils in den Teilern angeordnete Schlitze ineinander steckbar seien, so dass sich hier sogenannte Kreuzschlitzteiler ergeben. Diese Ausführungsvariante ist zwar vergleichsweise weniger flexibel in der Unterteilung, dafür handelt es sich aber um eine sehr stabile Ausführungsvariante.

Schließlich können auch im Bereich eines Teilers jeweils Bohrungen derart vorgesehen sein, dass die Steckclips auf diesen aufsteckbar sind. Gemäß dieser Ausführungsvariante sind die Steckclips nicht nur auf die Seitenwände aufsteckbar, sondern auch auf Teiler, da im Bereich dieser Teiler entsprechende Bohrungen im Wannenboden bzw. in Absätzen der Teiler vorgesehen sind. Im Bodenbereich könnten die Bohrungen beispielsweise auch durch auf den Wannenboden auflegbare Schienen die die entsprechenden Bohrungen enthalten, realisiert seien.

Gemäß der vorliegenden Erfindung können vergleichsweise dünnere Teiler verwendet werden. Während nach dem Stand der Technik üblicherweise Teiler aus Kunststoff mit einer Wandstärke von 5 mm zur Anwendung kommen, können aufgrund der erhöhten Stabilität der Halterung der Teiler die Materialstärken auf eine Wandstärke auf 3 mm reduziert werden.

Gemäß einem weiteren vorteilhaften Aspekt der Erfindung können Schiebeclip und Teiler aus verschiedenen Materialien hergestellt sein. So kann beispielsweise der Clip aus Kunststoff gespritzt sein, während der Teiler aus einem anderen hochwertigeren oder aber aus Kostengründen aus einem anderen minderwertigeren Kunststoff besteht. Bei erhöhten Festigkeitsanforderungen an den Teiler kann dieser beispielsweise auch aus Aluminium oder einem anderen Material höherer Festigkeit gefertigt werden.

Weitere Merkmale, Einzelheiten und Vorteile der Erfindung ergeben sich aus den in der Zeichnung dargestellten Ausführungsbeispielen. Es zeigen:
- Figur 1:: eine perspektivische Darstellung einer Wanne einer ersten Ausführungsvariante des Modul-Wannensystems nach der vorliegenden Erfindung,
- Figur 2:: einen Schnitt entlang eines Teils der Figur 1,
- Figur 3:: einen Steckclip, wie er in einer Ausführungsvariante gemäß Figur 1 eingesetzt wird, in Draufsicht, sowie drei Schnitte entlang der Schnittlinie A, der Schnittlinie B und der Schnittlinie C,
- Figur 4:: eine perspektivische Ansicht des Steckclips nach Figur 3,
- Figur 5:: eine perspektivische Darstellung einer Wanne einer zweiten Ausführungsvariante des Modul-Wannensystems gemäß der vorliegenden Erfindung,
- Figur 6:: die Darstellung gemäß Figur 5 in einer modifizierten Ausführungsform,
- Figur 7:: eine Schnittdarstellung durch einen Teil der Wanne gemäß Figur 5,
- Figur 8:: eine perspektivische Darstellung durch einen geschnittenen Teil der Figur 5,
- Figur 9:: eine weitere Ausführungsvariante einer Wanne gemäß Figur 5 und
- Figur 10:: wiederum eine weitere Ausführungsvariante einer Wanne gemäß dem Ausführungsbeispiel nach Figur 5.

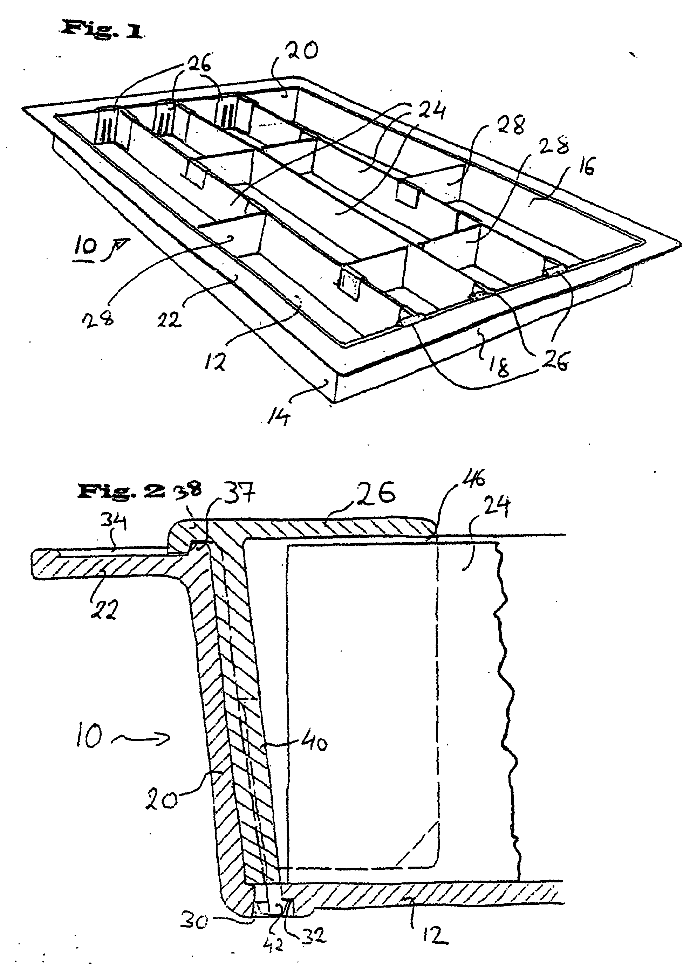

In den Figuren 1 bis 4 ist eine erste Ausführungsvariante des Modul-Wannensystems gezeigt. Das Modul-Wannensystem besteht zunächst aus einer Wanne 10, die im hier dargestellten Ausführungsbeispiel aus ABS (Acrylnitryl/Butadien/Styrolpolymer) gefertigt ist. Die Wanne weist einen Boden 12 und diesen umgebende Seitenwände 14, 16, 18 und 20 auf. Am oberen Rand der Seitenwände ist jeweils ein umlaufender Flansch 22 angeformt.

Wie aus der Figur 1 ersichtlich sind in der Figur 1 Teiler 24 über Steckclips 26 mit den Seitenwänden 18 und 20 verbunden. Hierdurch erhält die Wanne 10 eine Längsunterteilung. Im hier dargestellten Ausführungsbeispiel sind zur Querunterteilung auf den Teilern 24 senkrecht zu diesen ausgerichtete Schiebeteiler 28 vorgesehen. Die Schiebeteiler 28 sind als solches bereits bekannt und bedürfen hier keiner weiteren Erläuterung.

Den Figuren 2 bis 4 ist der genauere Aufbau des Steckclips 26 und die Art und Weise der Befestigung der Teiler 24 über die jeweiligen Steckclips 26 zu entnehmen. Im Schnitt gemäß Figur 2 ist ein Teil einer Wanne 10 mit einem Teil des Bodens 12 und der Seitenwand 20 zu entnehmen. Die Seitenwand verläuft, wie in Figur 2 gezeigt, nicht senkrecht zum Boden 12 sondern ist nach außen leicht angeschrägt. Im Übergang vom Boden 12 zur Seitenwand 20 ist eine Lochbohrung 30 gezeigt, die Teil einer über die gesamte Länge der Seitenwände verlaufenden Lochung ist. In der Bohrung ist jeweils ein Hinterschnitt 32 vorgesehen, wie in Figur 2 eingezeichnet. Der obere flanschartige Rand 22 weist eine Vertiefung 34 auf, so dass im unmittelbaren Randbereich der Wand 20 ein Vorsprung 37 gebildet wird.

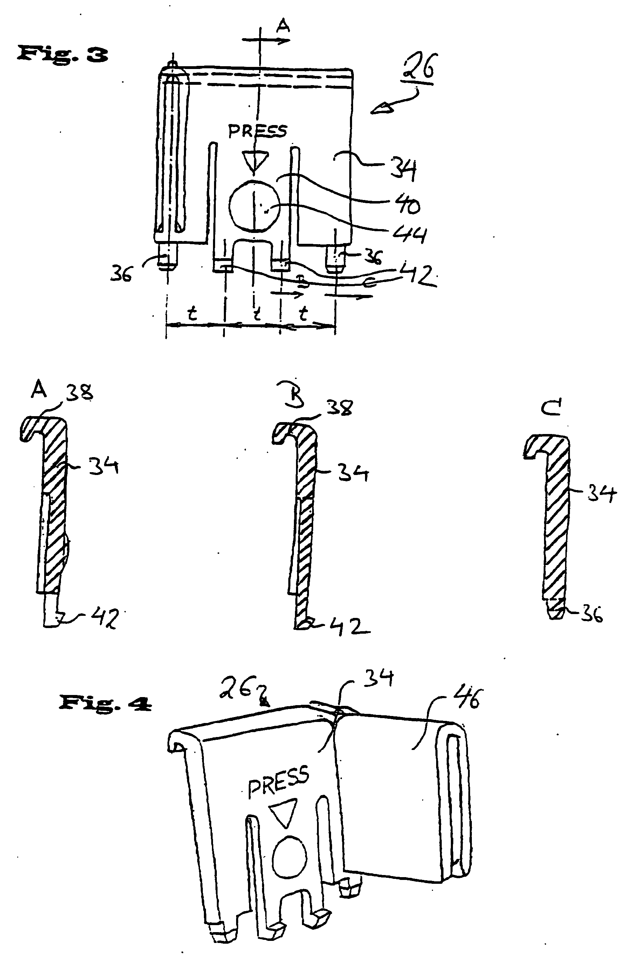

Aus Figur 3 ergibt sich der Aufbau des Steckclips 26. Dieser besteht aus einem im wesentlichen plattenförmigen Teil 34, das am unteren Ende einerseits zunächst zwei Steckstifte 36 aufweist, die in entsprechende Bohrungen 30 der Lochung im Bereich der Seitenwände 14, 16, 18 und 20 einsteckbar sind. Auf der gegenüberliegenden Seite ist ein den Vorsprung 37 übergreifender Bügel 38 angeformt. Mittig ist ein rastbarer Federmechanismus 40 in Form einer federnden Zunge ausgenommen, an dessen unteren Ende zwei Widerhaken 42 angeformt sind. Im Bereich der den rastbaren Federmechanismus 40 bildenden Zunge ist ein runder Vorsprung 44 angeformt, der für den Benutzer als Druckpunkt zur Betätigung des Federmechanismus, das heißt zum Entriegeln der Widerhaken 42, dient. Aus Figur 2 ist ersichtlich, wie der Steckclip 26 in der Seitenwand 20 eingesetzt ist. Mit seinem die Seitenwand 20 übergreifenden Bügel 38 ist der Steckclips 26 im oberen Bereich gehalten, während er über die eingesteckten Steckstifte 36 in den entsprechenden Bohrungen 30 der Lochung fixiert ist. Ein unbeabsichtigtes Herausrutschen aus der Bohrung 30 wird durch das Einrasten der Widerhaken 42 unter den jeweiligen Hinterschnitt 32 in der Bohrung verhindert. Durch entsprechendes Drücken auf den Vorsprung 44 kann der rastbare Federmechanismus 40 einfach wieder entriegelt werden, wenn der Steckclip mit dem Teiler 24 entfernt werden soll. Zum Festhalten des Teilers 24 weist der Steckclip 26 einen senkrecht auf die Platte 34 zulaufenden U-förmigen Bügel 46 auf. Dieser umklammert das Ende des Teilers 24 und hält diesen fest. Zur Sicherung des Teilers 24 in den U-förmigen Bügel 46 kann zusätzlich noch ein Stift, der durch eine Bohrung des Teilers verläuft, im U-förmigen Bügel 46 vorgesehen sein.

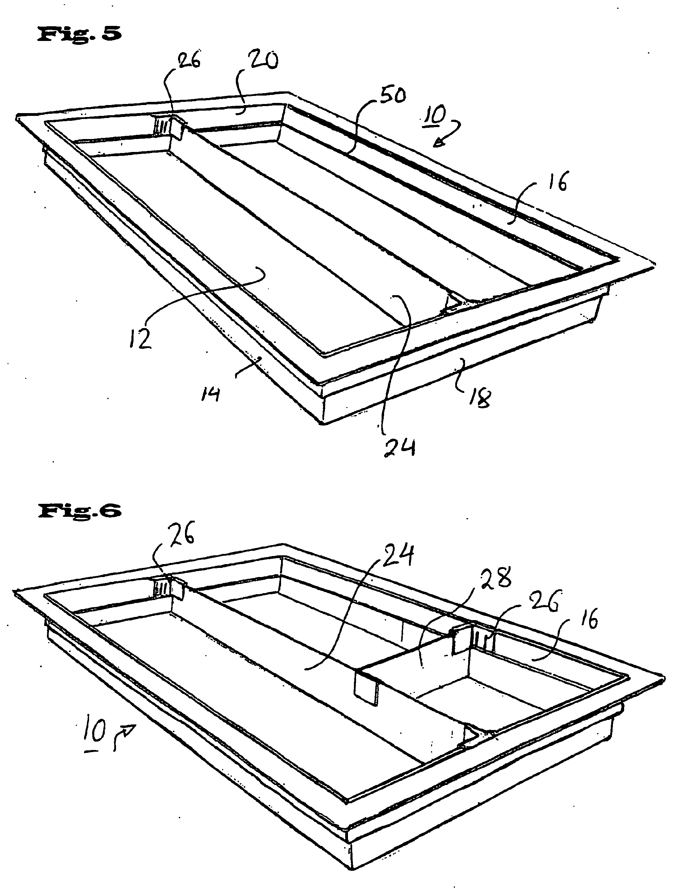

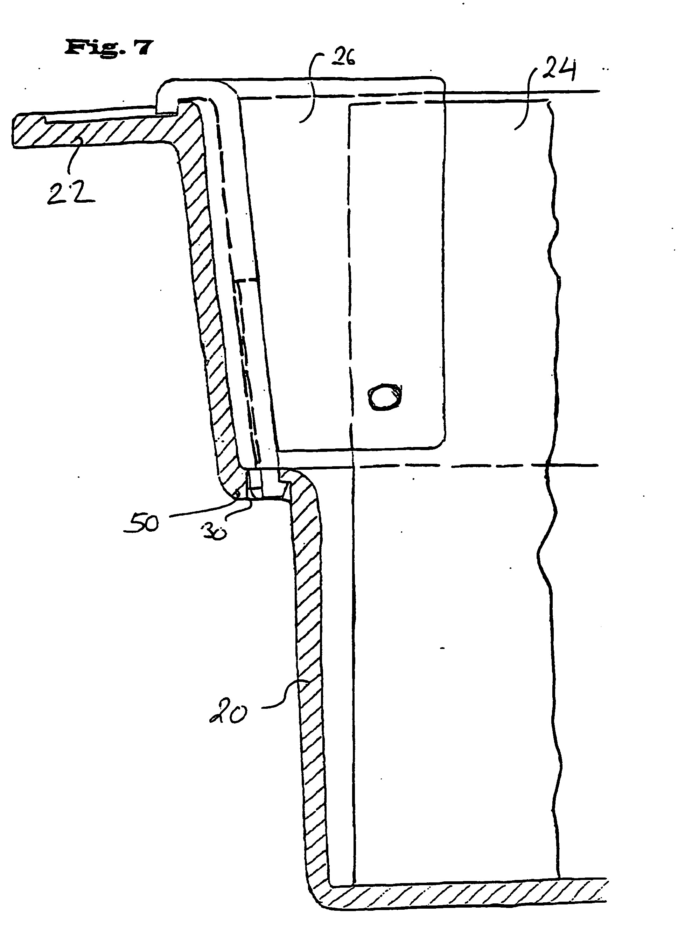

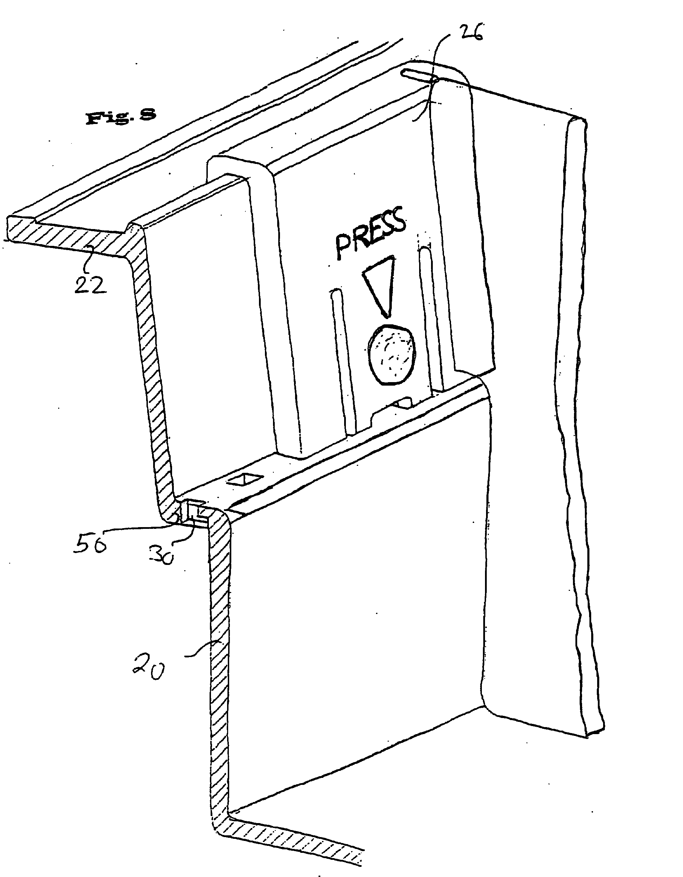

Anhand der Figuren 5 bis 10 werden alternative Ausgestaltungen entsprechender Wannen 10 dargestellt. Diese weiteren alternativen Ausführungsformen der Wannen betreffen jeweils Wannen mit einer Bauhöhe von 100 mm. Sie unterscheiden sich von der Ausführungsform gemäß Figur 1 dadurch, dass hier in der ansonsten glatten Seitenwandung 14, 16, 18, 20 jeweils ein Absatz 50 vorgesehen ist, in welchem die Lochung zur Aufnahme der Steckstifte der Steckclip 26 angeordnet sind. Hierdurch ergibt sich im unteren Randbereich zwischen Wandung 14, 16, 18 und 20 und dem Boden 12 eine dichte nicht gelochte Ausführung, so dass die Wannen in den Ausführungsformen der Figuren 5 bis 10 flüssigkeitsdicht sind. Der Absatz 50 ist ungefähr in der Hälfte der Wandhöhe also bei ca. 50 mm vorgesehen. Hierdurch können Steckteiler 26 einer einheitlichen Baugröße, wie sie auch bei den vergleichsweise niedriger bauenden Wannen 10 gemäß Figur 1 (mit einer Bauhöhe von 50 mm) einsetzbar sind, verwendet werden. Der Aufbau des Steckclips 26, wie er in den Figuren 7 und 8 dargestellt ist, entspricht demjenigen der Figuren 2 bis 4 und bedarf daher keiner weiteren Ausführung. Insbesondere den Figuren 7 und 8 ist zu entnehmen, dass die jeweilige Bohrung 30 hier im Absatz 50 vorgesehen ist. Während der Clip 26 die gleiche Größe aufweist wie in der Figur 2, ist in der Ausführungsform gemäß der Figur 7 der Teiler 24 doppelt so hoch wie derjenige gemäß der Figur 2.

Wie der Figur 7 zu entnehmen ist, weist die untere Seitenwand eine Schräge von 1,5° auf, während die Seitenwand 20 nach dem Absatz 50 eine Schräge von 5° aufweist. Bei bekannten Wannen wird über die gesamte Höhe der Seitenwandung eine Schräge von 5° eingehalten, um eine Stapelbarkeit zu gewährleisten. Eigentlich würde aus formtechnischen Gründen eine Entformungsschräge von 1,5° ausreichen. Die hier gezeigte Ausführung der Wanne mit einem Absatz 50 ermöglicht es nun, zumindest den unteren Teil der Seitenwand 20 lediglich mit der Entformungsschräge von 1,5° zu versehen. Die Stapelbarkeit ist hier durch das Vorsehen des Absatzes 50 gewährleistet. Aufgrund der zumindest im unteren Bereich der Seitenwand 20 realisierbaren kleineren Schräge von 1,5° statt 5° verbessert die Raumausnutzung der Wanne.

Die Figuren 6, 9 und 10 weisen nun unterschiedliche Möglichkeiten für die Ausführung von Querteilern auf. In der Ausführungsvariante gemäß Figur 6 ist eine Wanne 10 mit einem Teiler 24 dargestellt, der grundsätzlich demjenigen gemäß Figur 5 entspricht. Hier ist nun zusätzlich ein Querteiler 28 vorgesehen, der als Schiebeteiler auf dem Teiler 24 aufsitzt. An seinem freien Ende ist er aber zur Verbesserung der Stabilisierung über einen weiteren Steckclip 26 mit der Seitenwand 16 verbunden.

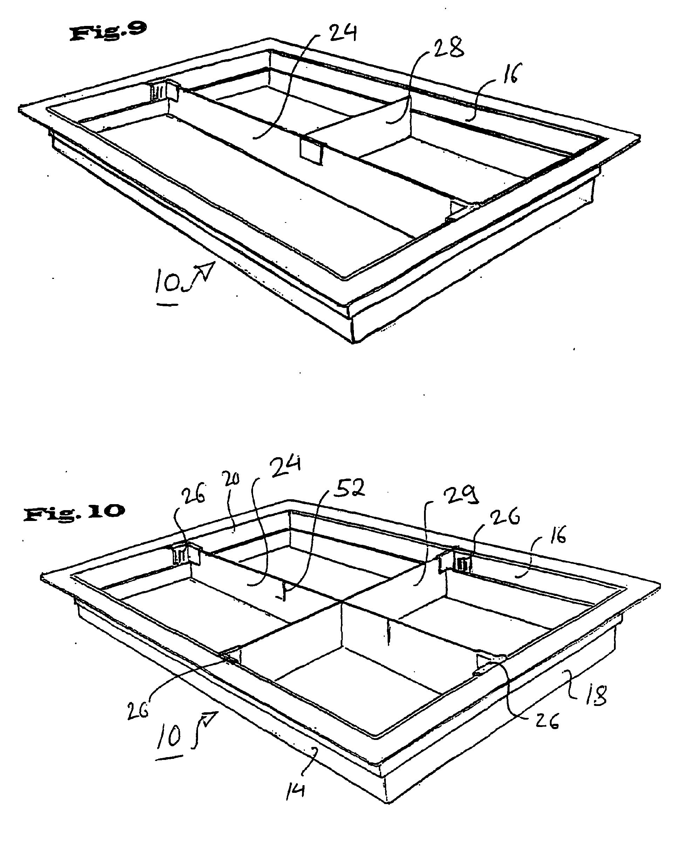

Dagegen ist in der Ausführungsvariante gemäß der Figur 9, die im Grunde ebenfalls derjenigen gemäß Figur 5 entspricht und einen Teiler 24 aufweist, auf dem Teiler 24 lediglich ein Schiebeteiler 28 aufgesetzt, dessen dem Teiler 24 gegenüberliegendes Ende nicht nochmals mit der Wand 16 verankert ist. Eine derartige Ausführungsvariante bietet sich für die Lagerung leichter Gegenstände in den entsprechenden sich bildenden Fächern an, während die Ausgestaltung gemäß Figur 6 für die Lagerung schwererer Gegenstände geeignet ist, da durch den entsprechenden Steckclip der Querteiler 28 verschiebefest in der Wanne 10 befestigt ist.

Gemäß der Ausgestaltung nach Figur 10 wiederum ist eine Wanne 10 der Bauform gemäß Figur 5 enthalten, in der der Teiler 24 ungefähr bis zur Hälfte verlaufende Einschnitte 52 aufweist, in welche Querteiler 29 mit ebenfalls entsprechend vorgesehenen Einschnitten einsteckbar sind, so dass sich hier eine entsprechende Kreuzteilung ergibt. Sowohl die Teiler 24 wie auch die Querteiler 29 sind durch Steckclips 26 mit den Wänden 14 bzw. 16 und 18 bzw. 20 verbunden.

## Patentansprüche

1. Modul-Wannensystem, insbesondere zur Lagerung von Medikamenten und Verbrauchsgütern für medizinische Zwecke, bestehend aus einer einen Boden (12) und Seitenwände (14, 16, 18, 20) aufweisenden Wanne (10) und in die Wanne (10) senkrecht zum Boden (12) verstellbar einsetzbaren Teilern (24), wobei die Teiler(24) jeweils mittels Steckclips (26) auf den jeweils glatt ausgeführten Seitenwänden (14, 16, 18, 20) gehalten sind, wobei jeder Steckclip (26) mindestens einen Steckstift (36) umfaßt, mit dem der Steckclip (26) in eine zugehörige Lochung (30) in der Wanne einsteckbar ist, wobei Steckclip (26) und Teiler (24) mehrteilig und zusammensteckbar ausgebildet sind
**dadurch gekennzeichnet,**
**dass** am Steckclip (26) ein U-förmiger Bügel (46) zur Aufnahme des Teilers (24) ausgebildet ist.

2. Modul-Wannensystem nach Anspruch 1, **dadurch gekennzeichnet, dass** im Steckclip (26) ein rastbarer Federmechanismus (40) integriert ist.

3. Modul-Wannensystem nach Anspruch 2, **dadurch gekennzeichnet, dass** der rastbare Federmechanismus (40) mindestens einen Widerhaken (42) umfaßt, der in der Lochung (30) verrastbar ist.

4. Modul-Wannensystem nach einem der Ansprüche 1 bis 3, **dadurch gekennzeichnet, dass** der Steckclip (26) einen die Seitenwand (14, 16, 18, 20) übergreifenden Bügel (38) aufweist.

5. Modul-Wannensystem nach einem der Ansprüche 1 bis 4, **dadurch gekennzeichnet, dass** an jedem Ende des Teilers (24) ein Steckclip (26) angeordnet ist.

6. Modul-Wannensystem nach Anspruch 1, **dadurch gekennzeichnet, dass** innerhalb des U-förmigen Bügels (46) mindestens ein Stift ausgebildet ist, der in aufgesteckter Position in mindestens eine entsprechende Bohrung evt. Schlitz im Teiler (24) eingreift.

7. Modul-Wannensystem nach einem der Ansprüche 1 bis 6, **dadurch gekennzeichnet, dass** die Lochung (30) im Boden (12) der Wanne (10) angeordnet ist.

8. Modul-Wannensystem nach einem der Ansprüche 1 bis 6, **dadurch gekennzeichnet, dass** die Lochung (30) in einem Absatz (50) der Seitenwandung oder Seitenwänden (14, 16, 18, 20) angeordnet ist.

9. Modul-Wannensystem nach einem der Ansprüche 1 bis 6, **dadurch gekennzeichnet, dass** die Lochung (30) im oberen Rand der Wanne (10) angeordnet ist.

10. Modul-Wannensystem nach einem der Ansprüche 3 bis 9, **dadurch gekennzeichnet, dass** in den jeweiligen Bohrlöchern ein Hinterschnitt zur Aufnahme der Rasthaken (42) des rastbaren Federmechanismus (40) des Steckclips (26) vorgesehen sind.

11. Modul-Wannensystem nach einem der Ansprüche 1 bis 10, **dadurch gekennzeichnet, dass** die Wanne (10) einen flanschartigen Rand (22) aufweist, der im Randbereich der Wanne (10) unter Ausbildung eines durch den Bügel (38) des Steckclips (26) übergreifbaren Randes nach unten zurückspringt.

12. Modul-Wannensystem nach einem der Ansprüche 1 bis 11, **dadurch gekennzeichnet, dass** die Wannen (10) im wesentlichen geschlossen ausgebildet sind.

13. Modul-Wannensystem nach einem der Ansprüche 1 bis 11, **dadurch gekennzeichnet, dass** die Wannen (10) Durchbrüche aufwesen und so einen Korb bilden.

14. Modul-Wannensystem nach einem der Ansprüche 1 bis 13, **dadurch gekennzeichnet, dass** in einer Wanne (10) mehrere parallel zueinander angeordnete Teiler (24) eingesetzt sind.

15. Modul-Wannensystem nach Anspruch 14, **dadurch gekennzeichnet, dass** auf den Teilern (24) senkrecht zu diesen ausgerichtete Schiebeteiler (28, 29) sitzen.

16. Modul-Wannensystem nach Anspruch 14, **dadurch gekennzeichnet, dass** senkrecht zu den Teilern (24) Teiler (28, 29) über jeweils in den Teilern (24) angeordnete Schlitze ineinander steckbar sind.

17. Modul-Wannensystem nach einem der Ansprüche 1 bis 16, **dadurch gekennzeichnet, dass** im Bereich eines Teilers (24, 28, 29) Bohrungen derart vorgesehen sind, dass die Steckclips (26) auf diesen aufsteckbar sind.

## Claims

1. Modular tray system, in particular for storing medicaments and consumer goods for medicinal purposes, comprising a tray (10) which has a base (12) and side walls (14, 16, 18, 20), and also comprising dividers (24) which can be inserted in an adjustable manner into the tray (10) perpendicularly to the base (12), the dividers (24) each being retained on the respectively smooth side walls (14, 16, 18, 20) by means of plug-in clips (26), each plug-in clip (26) comprising at least one plug-in pin (36) by means of which the plug-in clip (26) can be plugged into associated perforations (30) in the tray, and the plug-in clip (26) and divider (24) being designed in a number of parts such that they can be plugged together, **characterized in that** a U-shaped bracket (46) for accommodating the divider (24) is formed on the plug-in clip (26).

2. Modular tray system according to Claim 1,
**characterized in that** a latching-action spring mechanism (40) is integrated in the plug-in clip (26).

3. Modular tray system according to Claim 2,
**characterized in that** the latching-action spring mechanism (40) comprises at least one barb (42) which can be latched in the perforations (30).

4. Modular tray system according to one of Claims 1 to 3, **characterized in that** the plug-in clip (26) has a bracket (38) engaging over the side wall (14, 16, 18, 20).

5. Modular tray system according to one of Claims 1 to 4, **characterized in that** a plug-in clip (26) is arranged at each end of the divider (24).

6. Modular tray system according to Claim 1, **characterized in that** the U-shaped bracket (46) has formed within it at least one pin which, in the plugged-on position, engages in at least one corresponding bore or slot in the divider (24).

7. Modular tray system according to one of Claims 1 to 6, **characterized in that** the perforations (30) are arranged in the base (12) of the tray (10).

8. Modular tray system according to one of Claims 1 to 6, **characterized in that** the perforations (30) are arranged in a shoulder (50) of the side wall or side walls (14, 16, 18, 20).

9. Modular tray system according to one of Claims 1 to 6, **characterized in that** the perforations (30) are arranged in the upper periphery of the tray (10).

10. Modular tray system according to one of Claims 3 to 9, **characterized in that** the respective holes contain an undercut for accommodating the barbs (42) of the latching-action spring mechanism (40) of the plug-in clip (26).

11. Modular tray system according to one of Claims 1 to 10, **characterized in that** the tray (10) has a flange-like periphery (22) which yields downwards in the peripheral region of the tray (10) to form a periphery over which the bracket (38) of the plug-clip (26) can engage.

12. Modular tray system according to one of Claims 1 to 11, **characterized in that** the trays (10) are of essentially closed design.

13. Modular tray system according to one of Claims 1 to 11, **characterized in that** the trays (10) have through-passages and thus form a basket.

14. Modular tray system according to one of Claims 1 to 13, **characterized in that** a tray (10) has a plurality of dividers (24) arranged parallel to one another inserted into it.

15. Modular tray system according to Claim 14, **characterized in that** sliding dividers (28, 29) are seated on the dividers (24) perpendicularly thereto.

16. Modular tray system according to Claim 14, **characterized in that**, perpendicularly to the dividers (24), dividers (28, 29) can be plugged into one another via slots which are arranged in the dividers (24) in each case.

17. Modular tray system according to one of Claims 1 to 16, **characterized in that** bores are provided in the region of a divider (24, 28, 29) such that the plug-in clips (26) can be plugged onto the same.

## Revendications

1. Système de cuve modulaire, en particulier pour stocker des médicaments et des biens de consommation à des fins médicaux, constitué d'une cuve (10) présentant un fond (12) et des parois latérales (14, 16, 18, 20) et des séparateurs (24) insérables dans la cuve (10) perpendiculairement au fond (12) d'une manière ajustable, où les séparateurs (24) sont retenus, respectivement par des clips enfichables (26) sur les parois latérales respectivement lisses (14, 16, 18, 20), où chaque clip enfichable (26) comporte au moins une tige enfichable (36) au moyen de laquelle le clip enfichable (26) peut être inséré dans un perçage associé (30) dans la cuve, où le clip enfichable (26) et le séparateur (24) sont réalisés en plusieurs parties et de manière à pouvoir être assemblés, **caractérisé en ce qu'**est réalisé au clip enfichable (26) un étrier en forme de U(46) pour la réception du séparateur (24).

2. Système de cuve modulaire selon la revendication 1, **caractérisé en ce qu'**est intégré dans le clip enfichable (26) un mécanisme à ressort encliquetable (40).

3. Système de cuve modulaire selon la revendication 2, **caractérisé en ce que** le mécanisme à ressort encliquetable (40) comprend au moins un crochet (42) qui peut s'encliqueter dans le perçage (30).

4. Système de cuve modulaire selon l'une des revendications 1 à 3, **caractérisé en ce que** le clip enfichable (26) présente un étrier (38) passant sur la paroi latérale (14, 16, 18, 20).

5. Système de cuve modulaire selon l'une des revendications 1 à 4, **caractérisé en ce qu'**il est disposé à chaque extrémité du séparateur (24) un clip enfichable (26).

6. Système de cuve modulaire selon la revendication 1, **caractérisé en ce qu'**est réalisée à l'intérieur de l'étrier en forme de U(46) au moins une tige qui, en position enfichée, s'engage dans au moins un perçage éventuellement fente correspondante dans le séparateur (24).

7. Système de cuve modulaire selon l'une des revendications 1 à 6, **caractérisé en ce que** le perçage (30) est disposé dans le fond (12) de la cuve (10).

8. Système de cuve modulaire selon l'une des revendications 1 à 6, **caractérisé en ce que** le perçage (30) est ménagé dans un gradin (50) de la paroi latérale ou des parois latérales (14, 16, 18, 20).

9. Système de cuve modulaire selon l'une des revendications 1 à 6, **caractérisé en ce que** le perçage (30) est disposé dans la zone supérieure de la cuve (10).

10. Système de cuve modulaire selon l'une des revendications 3 à 9, **caractérisé en ce que** sont prévus dans les trous de perçage respectifs une contre-dépouille pour la réception des crochets d'encliquetage (42) du mécanisme à ressort encliquetable (40) du clip enfichable (26).

11. Système de cuve modulaire selon l'une des revendications 1 à 10, **caractérisé en ce que** la cuve (10) présente un bord en forme de bride (22) qui, dans la zone de bord de la cuve (10), en réalisant un bord pouvant passer sur l'étrier (38) du clip enfichable (26), rebondit vers le bas.

12. Système de cuve modulaire selon l'une des revendications 1 à 11, **caractérisé en ce que** les cuves (10) sont réalisées d'une manière sensiblement fermée.

13. Système de cuve modulaire selon l'une des revendications 1 à 11, **caractérisé en ce que** les cuves (10) présentent des perçages et forment ainsi un palier.

14. Système de cuve modulaire selon l'une des revendications 1 à 13, **caractérisé en ce que** sont placés dans une cuve (10) plusieurs séparateurs (24) disposés parallèlement les uns aux autres.

15. Système de cuve modulaire selon la revendication 14, **caractérisé en ce que** sont disposés sur les séparateurs (24) des séparateurs coulissants (28, 29) orientés perpendiculairement à ceux-ci.

16. Système de cuve modulaire selon la revendication 14, **caractérisé en ce que**, perpendiculairement aux séparateurs (24), les séparateurs (28, 29) peuvent être enfichés l'un dans l'autre par respectivement des fentes ménagées dans les séparateurs (24).

17. Système de cuve modulaire selon l'une des revendications 1 à 16, **caractérisé en ce que** sont prévus dans la zone d'un séparateur (24, 28, 29) des perçages de telle sorte que les clips enfichables (26) peuvent être enfichés sur celui-ci.
